# EUROPEAN PATENT SPECIFICATION

(11) **EP 0 561 838 B1**
(45) Date of publication and mention of the grant of the patent: **16.10.1996**
(21) Application number: 91920759.7
(22) Date of filing: 29.11.1991
(51) Int. Cl.: A61M 15/00

(54) **POWDERED MEDICAMENT DISPENSING DEVICE**
ABGABEVORRICHTUNG FÜR PULVERFÖRMIGE ARZNEISTOFFE
DISPOSITIF DISTRIBUTEUR DE MEDICAMENT EN POUDRE

(30) Priority: 15.12.1990 GB 9027234
(43) Date of publication of application: 29.09.1993
(73) Proprietor: NORTON HEALTHCARE LIMITED, Harlow Essex CM19 5TJ (GB)
(72) Inventor: BACON, Raymond 36 Down End Road, Portsmouth Hampshire PO6 1HU (GB)
(74) Representative: Green, Mark Charles
(86) International application number: PCT/GB91/02117
(87) International publication number: WO 92/10229

(56) References cited:
- DE-A- 4 004 904

## Description

This invention relates to a dispensing device which is suitable for the dispensing and administration of a metered amount of powder. Such a device would be suitable for the dispensing and administration of pure powder form drugs or drugs mixed with a suitable carrier agent e.g. lactose.

Metered dose inhalers are well known and often comprise a pressurised aerosol dispensing container. The aerosols contain gas, propellants in which the powdered medicament is suspended. On actuation, the aerosol contents are expelled, through a metering valve, and a metered dose is propelled into the lungs of the patient.

Research has indicated that some aerosol propellants, including those used in metered dose inhalers can cause depletion of the ozone layer in the atmosphere. It has thus become more important that such inhalers can be substituted with metered dose inhalers which do not have a damaging effect on the environment. Furthermore, such aerosol systems are not suitable for some patients.

Several types of powder inhalers are known. Usually a metered dose of medicament is initially contained in a container. The container is often in the form of a gelatin capsule. The capsule is first opened e.g. by piercing with a pin, and then its contents are dispersed and expelled by ensuring that airflow, due to the inhalation of the patient, causes the capsule to rotate.

These powder dispensers have several disadvantages. It is necessary for the patient to reload the dispenser after each dose release, and in some devices the capsules must be pierced before loading. Complicated mechanisms are employed to ensure complete expulsion of powder in order to provide the correct dose to the patient. This can make the devices difficult to operate and expensive to manufacture.

GB 2102295 and GB 2144997 disclose a complicated inhaler in which a metered dose of medicament is dispensed from a storage chamber containing powdered medicament in a pelletised micronised form. The inhaler includes a dosing unit which is connected to a storage chamber for the medicament. The dosing unit comprises a perforated rotating membrane, and spring loaded scrapers to fill the rotating perforations with medicament. The filled perforations are introduced to a passage which connects a propellant container to a nozzle. An amount of propellant is released when the patient depresses two triggers in succession. The propellant expels the contents of the exposed perforations towards the nozzle to be inhaled by the patient. The size of the metered dose is determined by the size of the perforations and the number of perforations that are brought into the propellant passage.

Such a device is expensive to manufacture, and the dosage accuracy relies on the efficiency of the scrapers to fill the perforations. The perforations often need to be presented several times to the powdered medicament to ensure complete filling. For optimum effect the device also requires the patient to coordinate inhalation with the operation of propellant release. Many patients find this coordination difficult to achieve.

EP 0069715 discloses a device which attempts to overcome some of the aforementioned problems. There is disclosed a powder inhaler which is actuated by she airflow generated by the inhalation of the patient. A breath actuated device eliminates the problem of the coordination of manual actuation and inhalation. A propellant is no longer necessary to effect actuation. The device also uses a perforated membrane and spring loaded scrapers to provide a metered dose of medicament. The patient rotates a manoeuvreing unit by a certain amount. This rotates the perforated membrane with respect to the scrapers filling the perforations and exposing a certain number of them to an air passage. The air flow generated on inhalation passes through the perforations and the metered dose is inhaled by the patient. A rotating means is provided to disrupt the airflow so as to break up any aggregate particles which have been formed in the dosing unit.

This device has the disadvantage that the airflow generated on inhalation passes directly through the perforations which are then returned to the dry storage chamber for refilling. Any powder which has become lodged in the perforations may become contaminated by the air, and this is then mixed with the pure dry powder held in the chamber. If the perforations become partially blocked then a full dose of medicament will not be inhaled by the patient.

It is the object of this invention to provide a metered dose powder inhaler, wherein the powdered medicament is stored in a powder reservoir housed in the device. It is a further object of the invention to provide such an inhaler which is simple in design yet overcomes the disadvantages experienced with prior art dispenses.

The present application relates to:
A powder inhalation device comprising a powder reservoir capable of containing a powdered medicament and a volume of air, a metering chamber extending from the powder reservoir to allow removal of the powdered medicament from the reservoir in discrete amounts, passage means to allow air to vent from the powder reservoir, through or across the metering chamber, and means for creating an air pressure difference between said reservoir and said passage means to cause an air flow from the powder reservoir through or across the metering chamber and into said passage means.

In a preferred arrangement the reservoir may be partly defined by a thin walled cylinder-like structure which interconnects with the main body of the device. The cylindrical reservoir and the main body may interconnect by way of a bore located in the main body of the device. It is preferred that the walls of the cylinder are in close sliding contact with the bore, whilst allowing air to pass from the reservoir, through the metering chamber and into the atmosphere.

In a further arrangement the metering chamber may be defined in the wall of the cylindrical reservoir. The chamber may comprise a hole in the wall of the powder reservoir. The hole is of a predetermined size to allow the desired dosage of powder to pass from the reservoir ready to be delivered to the main air conduit which enables the powdered medicament to be inhaled by a patient. The chamber may be sealed with a fine filter, or may be a depression in the wall with appropriate venting or leakage path provided. Certain hole shapes seem to give better metering repeatability as do different patterns of air leakage paths. Cylindrical chambers with their depth equal to the cylinder diameter are preferred.

It is preferred that the metering chamber is filled from the reservoir by the instigation of relative motion between the metering chamber and the powder bulk so that the air pressure on the powder bulk is increased, forcing the powder into the metering chamber whilst allowing air to pass in a small amount through the metering chamber and vent to atmosphere. Whilst in the device according to the present invention it is preferred that the powder front should pass across the entrance to the metering chamber by relative motion of the powder and the metering chamber, it has been found that the metering chamber will be filled even if the powder bulk is in contact with the entrance to the chamber and the air pressure above the powder bulk increased.

The relative motion may be provided by depression of the cylinder into a bore in the main body of the device, whilst the bulk of the powder is kept stationary by a protrusion positioned inside the bore. The cylinder may be depressed by the patient manually. Alternatively it may be depressed by the operation of a lever capable of acting on the cylinder to keep it depressed. It is preferred that the protrusion is provided with a seal, such that the inner bore of the cylinder and the protrusion seal are in sliding air tight contact.

The pressure entered on the powder bulk may be increased by compressing a volume of air contained above the powder. It will be realised that although reference herein has been made to air, any gas may be included within the reservoir which does not react with the powder, e.g. nitrogen.

Although not wishing to be bound by theory, it is likely that a local fluidising effect of the powder bulk may take place which aids metering, with the air flow through the chamber carrying powder into the chamber.

It will also be realised that since the metering effect seems to be caused primarily by air flow from the high pressure region within the air reservoir (and possibly powder) to a lower pressure region beyond the metering chamber, a similar effect could be created by producing a low pressure region outside the reservoir and metering chamber, and providing a pressure differential between the reservoir and this low pressure region to allow air flow. This is to be understood and included within the invention as claimed.

In a preferred arrangement, the metering chamber, once filled, is closed to separate the metered dose from the reservoir by causing the chamber to move past the protrusion located in the bore. The metered dose may be prevented from leaking into the bore in the main body by ensuring the cylinder walls and said bore are in close sliding contact, but spaced sufficiently to allow an air passage. The size of the air passage is linked to the powder particle size and should be sufficiently narrow that powder within the metering chamber will not escape. Many powdered drugs borne on carriers have an average particle diameter size of between 20 and 50 µm. Accordingly, the width of the air passageway is preferably from 10 to 100 µm, preferably 10 to 50 µm. The passageway can be achieved by utilising surface imperfections.

An exit port may be provided in the bore which, when aligned with the metering chamber, allows the chamber to be emptied. Once the powder has been metered it must then be ejected from the metering chamber. Clearly while more accurate volumetric metering is achieved with a higher packing density in the metering chamber, ejection of a more tightly packed powder is more difficult. The first part of this process requires isolation of the metering chamber from the bulk powder reservoir. This may be achieved by causing the reservoir volume to be moved away from the metering chamber until a sliding seal moves over (or over and past) the inner face of the metering chamber. In this embodiment the seal should be wide enough to prevent a leakage path occurring from the higher pressure reservoir via the metering chamber to a lower pressure area behind the sliding seal.

Ejection may take the form of increased air flow through the metering chamber carrying out the metered powder, air flow past the chamber, e.g. by a venturi-like restriction, creating a negative pressure to suck out the powder, or by mechanical ejection. Any air flow techniques could use the patient inspired air, but it is likely that dense packing and small metering chamber sizes may make this difficult particularly in terms of resistance to air flow. However, there is good potential during actuation in the specifically described form of the device to produce a small volume of air, at a pressure considerably greater or lower than atmospheric, ('high energy air') which can be utilised for dose ejection, and possibly dispersion. A further advantage of this technique is that inspired air does not come into direct contact with the metering chamber and surrounding walls. This could help prevent any risk of moisture contamination to the bulk powder from the inspired air contaminating the metering chamber and could be arranged to help avoid any such contamination risks should the patient breath out through the device.

Mechanical ejection techniques may preferably be combined with air flow techniques in order to remove powder which remains attached to the end of the ejector.

The metering chamber may also be in the form of a cup on a rotatable axis. Such a system is described in, for example, GB 2165159. If the metering chamber is a cup, the air will flow across the surface of the cup and powder will fill into the cup.

It is favoured that the air conduit allows air to enter an air inlet in the main body and flow to a mouthpiece, the air flow being caused by the inhalation of the patient. A venturi-type restriction and a secondary passageway may be included in the air conduit. The secondary passageway may connect the exit port to the restriction and further connect a secondary air inlet to the restriction. The air flow through the main inlet and secondary inlet transfers the metered dose to the patient for inhalation. Alternatively the air flow may be arranged to flow through the metering chamber.

The even distribution of the powder in the air flow before it is inhaled is preferably achieved by producing a turbulent air stream. This may be produced by including a swirl chamber in the air conduit.

It has been found that the most beneficial effect for patients is obtained when inspiration is carried out at at least 10 litres per minute, preferably at least 15 litres per minute. This may be achieved by incorporating a regulator within the inhalator to permit it to work only at a minimal air flow of, e.g. 10 litres per minute.

The present invention will be further described by way of example only, with reference to and illustrated in the accompanying drawings in which:-
Figure 1 is a section view according to an embodiment of the invention, in the rest position;
Figure 2 is a section view according to an embodiment of the invention in the actuated position.
Figure 3 is a section view of a further embodiment of the invention.

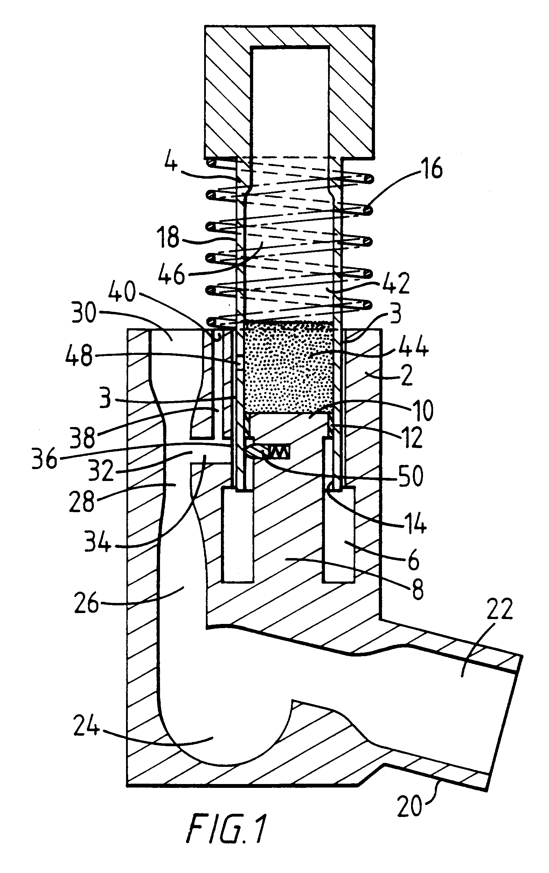

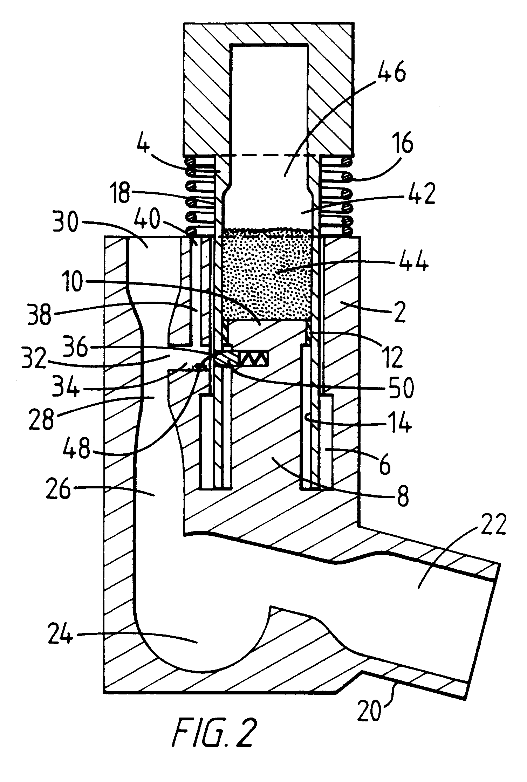

As seen in Figures 1 and 2 an inhalation device consists of a main body 2 and thin-walled cylinder-like chamber 4.

The main body 2 includes a bore 6 coaxial with the cylinder 4 and a protrusion which forms a piston structure 8 inside the bore 6. The piston 8 is also coaxial with the cylinder 4.

The piston head 10 is provided with a circumferential seal 12. The seal 12 ensures that the piston 8 is in slidable airtight contact with the inner bore 14 of the cylinder 4.

A passageway 3 is provided by a selection of surface finish to allow controlled venting to the atmosphere.

The cylinder 4 is free to move longitudinally in the bore 6 but is prevented from rotational movement [not shown]. A spring 16 is coiled coaxially around the cylinder 4. The cylinder walls 18 are in close sliding contact with the bore 6 separated by the air passageway 3. The spring 16 provides a means to bias the cylinder 4 in its rest position [shown in Figure 1].

The main body 2 has a mouthpiece 20 connected by a passageway 22 to a swirl chamber 24. The swirl chamber 24 is in turn connected to a passage 26 which includes a venturi-type restriction 28 leading to an air inlet 30.

A side entry 32 in the narrow section of the restriction 28 leads to a secondary passage 34. The secondary passage 34 is connected to the main bore 6 by an exit port 36.

The main body 2 further includes a small bore 38. The small bore connects with the secondary passage 34 and is vented at a secondary air inlet 40 close to the air inlet 30.

The inner bore 14 of the cylinder 4, the piston head 10 and the piston seal 12 cooperate together to form a dry reservoir 42. The reservoir 42 contains a bulk of finely powdered medicament 44. A volume of air 46 is trapped above the medicament 44.

The cylinder wall 18 is provided with a metering chamber 48 comprising a hole in the cylinder wall 18. The volume of the metering chamber 48 is such that the amount of medicament which can be contained in that volume is equivalent to one dose.

The metering chamber 48 is so positioned in the cylinder wall 18 that when the cylinder 4 is in its actuated position [shown in Figure 2] the metering chamber 48 is aligned with the exit port 36 in the main body 2.

The piston 8 is provided with a small spring loaded plunger 50. The plunger 50 is aligned with the centre line of the exit port 36. When the cylinder 4 is in the rest position [shown in Figure 1] the plunger is restrained from operation by the cylinder wall 18.

When the cylinder 4 is in the actuated position shown in Figure 2] the plunger is projected into the metering chamber 48.

The main body 2 of the dispensing device and the cylindrical structure 4 are preferably manufactured from a plastic such as polypropylene, acetal or moulded polystyrene. They may however be manufactured from metal or another suitable material.

The piston head seal 12 may be a seal of plastic such as PTFE, synthetic rubber or natural rubber. The seal 12 may be a cup or lip seal extending around the piston head 12.

In use the patient holds the device such that the cylinder 4 is located uppermost. The patient then shakes the device, whilst holding it vertically. The shaking aids the mixing of the powdered medicament and also ensures that the powder is deposited at the bottom of the cylinder 4 in contact with the piston head 10.

The patient depresses the top of the cylinder 4. The spring 16 becomes compressed and the cylinder 4 moves down the bore 6 inside the main body 2.

As the cylinder 4 moves down the metering chamber 48 passes through the bulk of the medicament 44. At the same time, the air in the space 46 is compressed the volume enclosed by the cylinder walls 18 the piston head 10 and the piston seal 12 decreases. A small amount of air flows through the powder bulk 44, through the metering chamber 48, through the passageway 3 and into the atmosphere.

The combined action of the movement of the metering chamber 48 through the bulk medicament 44, the increase in pressure on the medicament and the air flow results in the filling of the chamber 48 with a metered dose of medicament.

The width of the passageway 3 is such that no medicament leaks out of the chamber 48.

The patient depresses the cylinder 4 until it reaches the end of its travel. The patient then inhales whilst keeping the cylinder 4 depressed.

In the actuated position [shown in Figure 2] all the powdered medicament 44 except that in the metering chamber 48 is sealed in the volume defined by the cylinder walls 18, the piston head 10 and the piston seal 12.

When the cylinder 4 is fully depressed the metering chamber 48 is aligned with the exit port 36 in the main body 2 and the spring loaded plunger 50.

The plunger 50 is no longer restrained by the cylinder walls 18 and as it springs forward the powder in the metering chamber 48 is pushed into the passage 34 through the exit port 36. The plunger is restrained from further movement by suitable means (not shown).

The inhalation of the patient causes air to enter through the inlet 30. The air reaches the venturi-type restriction 28 and the narrowing of the inlet causes the air velocity to increase. The air pressure in the restriction 28 decreases as a result of the increase of velocity. The drop in pressure causes a further stream of air to enter through the small bore 38 which in turn causes the metered dose of medicament to be dragged into the main air stream flowing through the restriction 28.

The metered dose of medicament is carried in the air stream through the passage 26 into the swirl chamber 24.

The geometry of the swirl chamber 24 causes the air and the powder to follow a circular path. The turbulent air flow in the swirl chamber results in the dispersion of the powder in the air flow.

The particles are carried in the air stream through the passage 22 to the patient via the mouthpiece 20. The patient thus inhales air containing a metered dose of medicament.

After use the patient releases the cylinder 4 and it returns to the rest position under the influence of the spring 16. The cylinder is provided with a limiting end stop [not shown] to prevent the cylinder and main body from becoming detached. As the cylinder 4 rises the plunger 50 is caused to retract by the movement of the cylinder wall 18 and the specific shape of the plunger 50. The enclosed space 46 returns to its original volume and the trapped air is no longer compressed.

The device is ready for further use.

The inhalation device may be manufactured as a sealed unit, which is discarded when the level of the powdered medicament 44 falls below the level of the metering chamber 48.

Alternatively the reservoir may be refilled through an opening in the top of the cylinder 4 which is normally sealed by a plug (not shown).

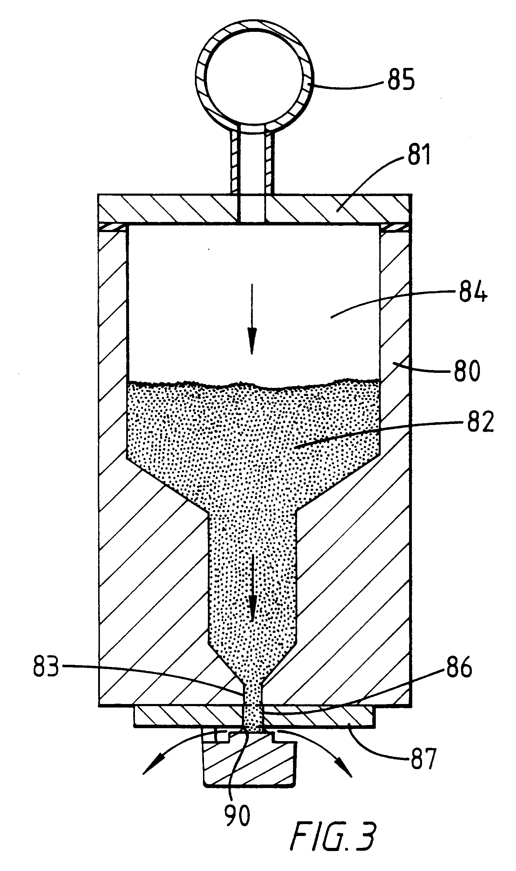

In a further embodiment of the device, as seen in Figure 3, an inhalation device consists of a chamber 80 which will be within the main body of the device [not shown]. A volume of powder 82 is included within the chamber 80. Above the powder 82 is a space 84 which is connected to a means 85 of increasing the pressure of the air within the space 84.

An orifice 83 leads from the chamber 80 into a metering chamber 86. This metering chamber 86 is formed in a plate 87 which is moveable relative to the body enclosing the chamber 80, in particular the orifice 83. Remote from the nozzle is an air gap 90.

As pressure is increased within the space 84, powder flows through the orifice 83 into the metering chamber 86. At the same time, air flows from the space 84 through the powder 82, through the orifice 83 and metering chamber 86 and out through the air gap 90 which is of such a size to prevent powder leakage. The metering chamber 86 is completely filled with powder.

The plate 87 is then slid sideways and the chamber 80 containing the metered dose of powder presented to the dispersion system. In order to refill the chamber, an airtight removeable lid 81 is provided.

Suitable drugs which may be used include salbutamol, beclomethasone dipropionate, budesonide and sodium cromoglycate.

## Claims

1. A powder inhalation device comprising a powder reservoir (42,80) capable of containing a powdered medicament (44,82) and a volume of air, a metering chamber (48,86) extending from the powder reservoir (42,80) to allow removal of the powdered medicament from the reservoir in discrete amounts, passage means to allow air to vent from the powder reservoir, through or across the metering chamber, and means (4,85) for creating an air pressure difference between said reservoir (42,80) and said passage means (3,90) to cause an air flow from the powder reservoir through or across the metering chamber (48,86) and into said passage means.

2. An inhalation device as claimed in Claim 1 wherein said metering chamber (48,86) is provided in a dose delivery structure, movable between a loading position in which the metering chamber communicates with the powder reservoir (42,80) and a delivery position in which the metering chamber communicates with a powder dispersion system.

3. An inhalation device as claimed in Claim 2 wherein the dose delivery structure is a slidably mounted plate member (87).

4. A powder inhalation device as claimed in any one of claims 1 to 3 comprising a means (4,85) for compressing the air in the reservoir wherein air vents into atmosphere as the pressure of the air in the powder reservoir is increased.

5. An inhalation device as claimed in any one of Claims 1 to 4 wherein the reservoir is housed in a thin-walled substantially cylindrical structure (4) and the metering chamber (48) is in the form of a hole defined in the wall of the powder reservoir (42).

6. An inhalation device as claimed in Claim 5 wherein the hole (48) is in the form of a cylindrical chamber having a depth substantially equal to the diameter of the cylinder.

7. An inhalation device as claimed in any one of the preceding Claims wherein as the metering chamber (48) is filled from the reservoir the bulk of the powder passes across the internal entrance to the metering chamber (48).

8. An inhalation device as claimed in Claim 7 wherein the reservoir is in sliding contact within a bore in the device, said bore having a protrusion (8) positioned therewithin, the powder being held within the reservoir by the protrusion.

9. An inhalation device as claimed in Claim 7 or Claim 8 wherein the metering chamber, once filled with powder, is closed to separate the metered dose from the reservoir by causing the chamber to move past the protrusion (8) located in the bore.

10. An inhalation device as claimed in any one of the preceding Claims wherein ejection of powder contained within the metering chamber is effected by air flow past the chamber.

11. An inhalation device as claimed in any one of Claims 1 to 9 wherein ejection of powder contained within the metering chamber (48) is effected by mechanical ejection (50).

12. An inhalation device as claimed in Claim 10 wherein the powder is ejected by positive or negative pressure caused by air flow.

13. An inhalation device as claimed in Claim 12 wherein negative pressure is caused by air flow through a venturilike restriction (28).

14. An inhalation device as claimed in Claim 1 or Claim 2 wherein the metering chamber is in the form of a cup.

## Patentansprüche

1. Pulverinhalations-Vorrichtung umfassend ein Pulverreservoir (42,80), das ein pulverförmiges Medikament (44,82) und ein Luftvolumen fassen kann, eine Dosierkammer (48,86), die sich von dem Pulverreservoir (42,80) erstreckt, um die Entnahme des pulverförmigen Medikaments aus dem Reservoir in diskreten Mengen zu ermöglichen, eine Durchgangseinrichtung, die den Abzug von Luft aus dem Pulverreservoir durch oder über die Dosierkammer ermöglicht und eine Einrichtung (4,85) zur Erzeugung einer Luftdruckdifferenz zwischen dem Reservoir (42,80) und der Durchgangseinrichtung (3,90), durch die ein Luftstrom von dem Pulverreservoir durch oder über die Dosierkammer (48,86) in die Durchgangsvorrichtung hervorgerufen wird.

2. Inhalationsvorrichtung gemäß Anspruch 1, wobei die Dosierkammer (48,86) als eine zwischen einer Füllstellung, in der die Dosierkammer mit dem Pulverreservoir (42,80) in Verbindung steht, und einer Abgabestellung, in der die Dosierkammer mit einem Pulverzerstreuungssystem in Verbindung steht, bewegliche Vorrichtung zur Abgabe der Dosis ausgebildet ist.

3. Inhalationsvorrichtung gemäß Anspruch 2, in der die Vorrichtung zur Abgabe der Dosis ein verschiebbar montiertes Plattenelement (87) ist.

4. Pulverinhalations-Vorrichtung gemäß einem der Ansprüche 1 bis 3, umfassend eine Vorrichtung (4,85) zum Komprimieren der in dem Reservoir befindlichen Luft, wobei Luft in die Atmosphäre austritt, wenn der Luftdruck in dem Pulverreservoir erhöht wird.

5. Inhalationsvorrichtung gemäß einem der Ansprüche 1 bis 4, wobei das Reservoir in einem dünnwandigen, im wesentlichen zylindrischen Aufbau (4) eingefügt ist und die Dosierkammer (48) in Form eines in der Wand des pulverreservoirs (42) definierten Loches vorgesehen ist.

6. Inhalationsvorrichtung gemäß Anspruch 5, wobei das Loch (48) die Form einer zylindrischen Kammer besitzt, deren Tiefe im wesentlichen dem Durchmesser des Zylinders entspricht.

7. Inhalationsvorrichtung gemäß einem der vorhergehenden Ansprüche, wobei, wenn die Dosierkammer (48) aus dem Reservoir befüllt wird, der Großteil des Pulvers über den inneren Eingang in die Dosierkammer (48) tritt.

8. Inhalationsvorrichtung gemäß Anspruch 7, wobei das Reservoir verschiebbar und in Kontakt mit einer in der Vorrichtung vorgesehenen Bohrung angeordnet ist, wobei innerhalb der Bohrung ein Vorsprung (8) vorgesehen ist, durch den das Pulver in dem Reservoir gehalten wird.

9. Inhalationsvorrichtung gemäß Anspruch 7 oder 8, wobei die Dosierkammer, sobald sie mit Pulver gefüllt ist, zur Abtrennung der bemessenen Dosis von dem Reservoir geschlossen wird, indem die Kammer dazu veranlaßt wird, sich hinter den in der Bohrung vorgesehenen Vorsprung (8) zu bewegen.

10. Inhalationsvorrichtung gemäß einem der vorhergehenden Ansprüche, wobei der Ausstoß von in der Dosierkammer enthaltenem Pulver durch einen Luftstrom hinter der Kammer bewirkt wird.

11. Inhalationsvorrichtung gemäß einem der Ansprüche 1 bis 9, wobei der Ausstoß von in der Dosierkammer (48) enthaltenem Pulver durch einen mechanischen Auswurf (50) bewirkt wird.

12. Inhalationsvorrichtung gemäß Anspruch 10, wobei das Pulver durch Über- und Unterdruck, hervorgerufen durch einen Luftstrom, ausgestoßen wird.

13. Inhalationsvorrichtung gemäß Anspruch 12, wobei durch einen Luftstrom durch eine Venturi-artige Verengung (28) ein Unterdruck erzeugt wird.

14. Inhalationsvorrichtung gemäß Anspruch 1 oder 2, wobei die Dosierkammer in Form eines Bechers ausgebildet vorgesehen ist.

## Revendications

1. Un dispositif d'inhalation de poudre comprenant un réservoir de poudre (42,80) capable de contenir un médicament en poudre (44,82) et un volume d'air, une chambre de dosage (48,86) s'étendant depuis le réservoir de poudre (42,80) pour permettre de prélever dans le réservoir des quantités discrètes de médicament en poudre des moyens de passage pour permettre à l'air de sortir du réservoir de poudre par passage à travers la chambre de dosage, et des moyens (4,85) pour créer une différence de pression d'air entre ledit réservoir (42,80) et lesdits moyens de passage (3,90) pour créer un courant d'air depuis le réservoir de poudre avec passage à travers la chambre de dosage (48,86) et dans lesdits moyens de passage.

2. Un dispositif d'inhalation selon la revendication 1, dans lequel ladite chambre de dosage (48,86) est pourvue d'une structure de distribution de dose, mobile entre une position de chargement dans laquelle la chambre de dosage communique avec le réservoir de poudre (42,80) et une position de distribution dans laquelle la chambre de dosage communique avec un système de dispersion de poudre.

3. Un dispositif d'inhalation selon la revendication 2, dans lequel la structure de distribution de dose est un organe en forme de plaque (87) monté coulissant.

4. Un dispositif d'inhalation selon l'une quelconque des revendications 1 à 3, comprenant des moyens (4,85) pour comprimer l'air dans le réservoir de telle manière que l'air s'échappe dans l'atmosphère lorsque la pression de l'air augmente dans le réservoir de poudre.

5. Un dispositif d'inhalation selon l'une quelconque des revendications 1 à 4, dans lequel le réservoir est logé dans une structure sensiblement cylindrique (4) à parois minces et la chambre de dosage (48) est sous la forme d'un trou défini dans la paroi du réservoir de poudre (42).

6. Un dispositif d'inhalation selon la revendication 5, dans lequel le trou (48) est sous la forme d'une chambre cylindrique dont la profondeur est sensiblement égale au diamètre du cylindre.

7. Un dispositif d'inhalation selon l'une quelconque des revendications précédentes dans lequel, lorsque la chambre de dosage (48) est remplie à partir du réservoir, l'ensemble de la poudre passe à travers l'entrée interne dans la chambre de dosage (48).

8. Un dispositif d'inhalation selon la revendication 7, dans lequel le réservoir est en contact de glissement dans un alésage du dispositif, ledit alésage comportant une partie en saillie (8) positionnée à l'intérieur, la poudre étant maintenue dans le réservoir par la partie en saillie.

9. Un dispositif d'inhalation selon la revendication 7 ou la revendication 8, dans lequel la chambre de dosage, une fois remplie de poudre est fermée pour séparer du réservoir la dose mesurée, en obligeant la chambre à se déplacer au-delà de la partie en saillie (8) située dans l'alésage.

10. Un dispositif d'inhalation selon l'une quelconque des revendications précédentes, dans lequel l'éjection de la poudre contenue dans la chambre de dosage est effectuée par l'air s'écoulant à travers la chambre.

11. Un dispositif d'inhalation selon l'une quelconque des revendications 1 à 9 dans lequel l'éjection de la poudre contenue dans la chambre de dosage (48) est effectuée par éjection mécanique (50).

12. Un dispositif d'inhalation selon la revendication 10 dans lequel la poudre est éjectée par une surpression ou une dépression provoquée par l'écoulement d'air.

13. Un dispositif d'inhalation selon la revendication 12 dans lequel la dépression est provoquée par un écoulement d'air à travers un étranglement (28) en forme de venturi.

14. Un dispositif d'inhalation selon la revendication 1 ou la revendication 2 dans lequel la chambre de dosage est en forme de coupe ou de cloche.
